# EUROPÄISCHE PATENTSCHRIFT

(11) **EP 1 198 445 B1**
(45) Veröffentlichungstag und Bekanntmachung des Hinweises auf die Patenterteilung: **22.01.2003**
(21) Anmeldenummer: 00952980.1
(22) Anmeldetag: 29.06.2000
(51) Int. Cl.: C07C 51/46, C07C 51/44, C07C 51/48, C07C 53/02, C07C 53/08

(54) **VERFAHREN ZUR TRENNUNG UND REINIGUNG EINES WÄSSRIGEN GEMISCHES AUS DEN HAUPTKOMPONENTEN ESSIGSÄURE UND AMEISENSÄURE**
A METHOD FOR THE SEPARATION OF AND PURIFICATION OF AN AQUEOUS MIXTURE CONSISTING OF THE MAIN COMPONENTS ACETIC ACID AND FORMIC ACID
PROCEDE DE SEPARATION ET DE PURIFICATION D'UN MELANGE AQUEUX COMPRENANT POUR PRINCIPAUX CONSTITUANTS DE L'ACIDE ACETIQUE ET DE L'ACIDE FORMIQUE

(30) Priorität: 22.07.1999 DE 19934411
(43) Veröffentlichungstag der Anmeldung: 24.04.2002
(73) Patentinhaber: Consortium für elektrochemische Industrie GmbH, 81379 München (DE)
(72) Erfinder: RÜDINGER, Christoph, D-82319 Starnberg (DE); VOIT, Harald, Herbert, D-84571 Reischach (DE); HALLMANN, Michael, A-5122 Hochburg-Ach (AT); GÜNALTAY, Mehmet, D-84547 Emmerting (DE); REIL, Barbara, D-84547 Emmerting (DE); EBERLE, Hans-Jürgen, D-81477 München (DE)
(74) Vertreter: Rimböck, Karl-Heinz, Dr.
(86) Internationale Anmeldenummer: EP0006083
(87) Internationale Veröffentlichungsnummer: WO01007390

(56) Entgegenhaltungen:
- EP-A- 0 732 320
- GB-A- 727 078
- GB-A- 788 931

## Beschreibung

Die Erfindung betrifft ein Verfahren zur Trennung und Reinigung eines wäßrigen Reaktionsgemisches aus den Hauptkomponenten Essigsäure und Ameisensäure.

Bei der Herstellung von Essigsäure durch katalytische Oxidationsreaktionen von gesättigten und/oder ungesättigten Kohlenwasserstoffen wie beispielsweise der Gasphasenoxidation von C₄-Kohlenwasserstoffen, fallen Produktströme an, die als Hauptkomponenten Essigsäure, Ameisensäure und Wasser in unterschiedlichen Zusammensetzungen enthalten.

Zur weiteren Aufarbeitung müssen diese Produktströme in ihre Einzelkomponenten getrennt werden. Eine destillative Trennung eines ternären Säure-Wasser Gemisches aus Essigsäure, Ameisensäure und Wasser in seine Reinkomponenten bereitet beispielsweise erhebliche Probleme, da das System neben dem binären Wasser-Ameisensäure-Maximumazeotrop zusätzlich ein ternäres Wasser-Ameisensäure-Essigsäure-Sattelazeotrop enthält.

Enthält ein derartiges Gemisch eine hohe Wasserkonzentration, so ergibt sich bei der destillativen Trennung ein enormer zusätzlicher Energiebedarf, da das gesamte Wasser als niedrigstsiedende Komponente über den Kopf einer Kolonne destilliert werden muß.

Hunsmann und Simmrock (Chemie-Ing.-Tech., 38, 1966) empfehlen zur Trennung von wäßrigen Gemischen mit einem Essigsäuregehalt von größer 60 Gew.-% und einem Ameisensäuregehalt von 5 Gew.-% die Anwendung der Azeotropdestillation zur Erleichterung der Trennung und zur Reduzierung der dabei benötigten Energie. Als Azeotropschleppmittel für die Entwässerung wird Ethyl-n-Butylether vorgeschlagen. Das Azeotrop aus Wasser und Schleppmittel siedet bei ca. 91°C und enthält etwa 10 Gew.-% Wasser. Das Schleppmittel Ethyl-n-Butylether bildet dabei kein Azeotrop mit Ameisensäure und Essigsäure.

In DE-A 1204214 wird zur Abtrennung von Ameisensäure die Azeotroprektifikation mit n-Butylchlorid als Schleppmittel empfohlen. Nachteilig an diesem Verfahren ist die Verwendung von chlorierten Kohlenwasserstoffen als Schleppmittel.

Aus US-A 5633402 ist ein Verfahren zur Trennung von binären Gemischen aus Ameisensäure und Essigsäure mittels Azeotropdestillation bekannt. Als Schleppmittel für die Ameisensäure wird dabei Methylformat verwendet. Eine Abtrennung von Wasser wird in diesem Verfahren nicht beschrieben.

Aus DE-A 4426132, EP-A 0635474, DE-A 19610356 (US-A 5662780), sind verschiedene Verfahren zur Reinigung und zur Entwässerung von Essigsäure mittels Azeotropen mit unterschiedlichen Schleppmitteln bekannt. Keines dieser Verfahren beschreibt jedoch die Entwässerung eines Gemisches aus Essigsäure und Ameisensäure.

Aus US-A 5173156, US-A 5006205, US-A 4877490 und US-A 4935100 sind Verfahren zur Entwässerung von Ameisensäure mittels Extraktivrektifikation bekannt. Dabei werden als Schleppmittel beispielsweise Cyclohexanon, Oxalsäure, Decansäure und Methylsalicylat genannt.

EP-A 156309 (CA-A 1238919) und EP-A 12321 (US-A 4262140) beschreiben die Entwässerung von Ameisensäure über Extraktivrektifikation mit Carboxamiden als Hilfsstoffe. Keines dieser Verfahren beschreibt jedoch die Entwässerung eines Gemisches aus Essigsäure und Ameisensäure.

Aus dem "Process Economics Program" Report No. 37A (1973) des Stanford Research Institute ist ein Verfahren zur Trennung eines wäßrigen Gemisches aus etwa 42 Gew.-% Essigsäure und 2 Gew.-% Ameisensäure bekannt. Das wäßrige Gemisch wird dabei durch Gegenstromextraktion mit Diisopropylether aufkonzentriert. In der Entwässerungs- und Lösungsmittelrückgewinnungskolonne wird das Wasser als Azeotrop aus Wasser und Diisopropylether über Kopf abdestilliert. Das Sumpfprodukt, ein Gemisch aus Essigsäure und Ameisensäure mit ca. 0,12 Gew.-% Wasser wird durch Azeotroprektifikation weiter aufgetrennt. Als Schleppmittel für die Ameisensäure wird Benzol verwendet. Von großem Nachteil an diesem Verfahren ist die geringe Qualität der abgetrennten Ameisensäure, die noch ca. 1 Gew.-% Essigsäure, ca. 2 Gew.-% Wasser und ca. 7 Gew.-% Benzol enthält. Die Verwendung von Benzol in diesem Verfahren und der Restgehalt an Benzol in der Ameisensäure machen dieses Verfahren jedoch unattraktiv.

Alle im Stand der Technik bekannten Verfahren sind entweder nur dazu geeignet binäre Mischungen wie Essigsäure/Wasser, Ameisensäure/Wasser, und Essigsäure/Ameisensäure zufriedenstellend zu trennen, oder nur für wäßrige Säuregemische wirtschaftlich anwendbar, in denen eine hohe Konzentration an Säure (> 60 Gew.-%) vorliegt. Weiterhin sind einige der bekannten Verfahren durch Ihren Einsatz von Benzol oder chlorierten Kohlenwasserstoffen nach heutigen Sicherheitsund Umweltgesichtspunkten nicht mehr akzeptabel.

Es bestand daher die Aufgabe ein Verfahren zur Trennung eines ternären, wäßrigen Gemisches aus Säuren in seine Reinkomponenten bereitzustellen, daß die im Stand der Technik genannten Nachteile nicht besitzt.

Es wurde nun gefunden, daß die Trennung und Reinigung eines Gemisches aus den Hauptkomponenten Essigsäure, Ameisensäure, Wasser und Hochsiedern (im weiteren Rohsäure genannt) besonders gut durchführbar ist, wenn in einem ersten Schritt mittels eines Lösungsmittels in einem Kreisverfahren extrahiert wird und anschließend der Extraktstrom, überwiegend bestehend aus Lösungsmittel, Essigsäure, Ameisensäure, Hochsiedern und Wasser, in einer Folge von Destillationsschritten in die Bestandteile Lösungsmittel, das zur Extraktion zurückgeführt wird, Wasser, Ameisensäure, Essigsäure und Hochsieder aufgetrennt wird, und der Raffinatstrom in einem weiteren Destillationsschritt mittels einer Lösungsmittelstripperkolonne vom Lösungsmittel befreit wird.

Gegenstand der Erfindung ist ein Verfahren zur Trennung und Reinigung eines wäßrigen Gemisches aus den Hauptkomponenten Essigsäure, Ameisensäure und Schwersiedern durch Extraktion, mittels eines Lösungsmittels in einem Kreisverfahren, dadurch gekennzeichnet, daß der Raffinatstrom mit einem Großteil des Wassers einer Lösungsmittelstripperkolonne (11) zur Auskreisung des Wassers zugeführt wird und der Extraktstom in eine Lösungsmitteldestillationskolonne (8) geleitet wird, aus der in einem ersten Schritt über Kopf eine Mischung (A) mit einem Großteil des Lösungsmittels (Leitung (1)), aus einem Seitenabzug eine Mischung (B) bestehend aus Ameisensäure, Wasser und Lösungsmittel und über den Sumpf eine Mischung (C) bestehend aus Essigsäure und Hochsiedern abgetrennt werden, und zur weiteren Verarbeitung die Mischung (B) in eine Ameisensäuredestillationskolonne (4) übergeführt wird (Leitung (2)), und die Mischung (C) in eine Essigsäuredestillationskolonne (5) übergeführt wird (Leitung (3)), anschließend in der Essigsäuredestillationskolonne (5) über Kopf die reine Essigsäure isoliert wird, in der Ameisensäuredestillationskolonne (4) am Sumpf die reine Ameisensäure isoliert wird und über Kopf ein Gemisch aus Lösungsmittel und Wasser abgezogen wird, welches zusammen mit der Mischung (A) nach Abtrennung des Wasseranteils, in den Extraktor (7) zurückgeführt wird.

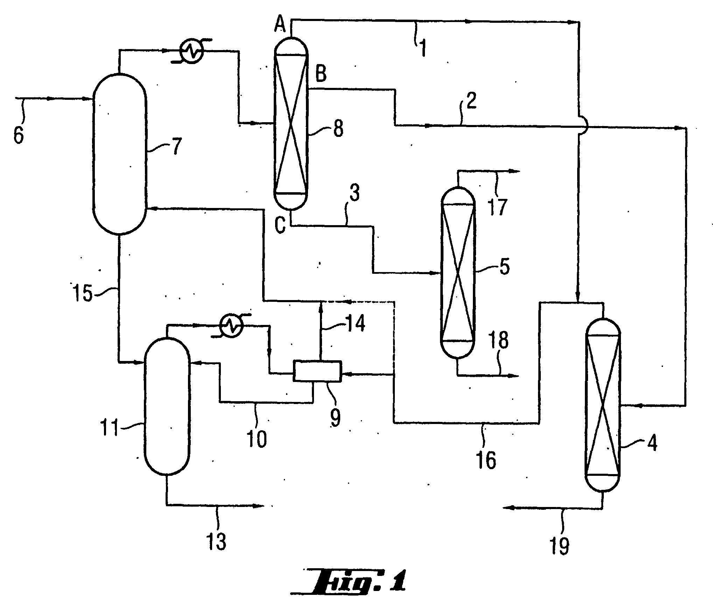

In der ersten Stufe (Extraktion) des erfindungsgemäßen Verfahrens (Fig. 1) wird die eingesetzte Rohsäure, bestehend aus wechselnden Anteilen von Essigsäure, Ameisensäure, Wasser und Schwersiedern einem Extraktor (7) zugeführt und mit einem Lösungsmittel kontaktiert. Der Extraktor (7) kann dabei einstufig oder bevorzugt mehrstufig aufgebaut sein. Der Lösungsmittelstrom kann in diesem Verfahren in Richtung des Stroms der Rohsäure gerichtet sein oder bevorzugt im Gegenstrom zur Rohsäure ausgelegt sein. Als Lösungsmittel können dabei Ether, Ester, Ketone, Alkohole, gesättigte, ungesättigte und cyclische Kohlenwasserstoffe mit 4 bis 8 Kohlenstoffatomen und deren Mischungen, bevorzugt Ether und Ester mit 4 bis 7 Kohlenstoffatomen, besonders bevorzugt Methyltertiärbutylether, Diisopropylether, Di-n-propoylecher, Ethylbutylether, Ethylacetat und Isopropylacetat, in einem Mischungsverhältnis zur Rohsäure zwischem dem 0,5 bis 20 fachen, bevorzugt 1 bis 5 fachen, besonders bevorzugt 1,5 bis 3,5 fachen (Verhältnis Volumen/Volumen) verwendet werden. Die Extraktion kann in einem Temperatur- und Druckbereich stattfinden, in dem das Extraktionslösungsmittel und die Rohsäure in flüssiger Form und als getrennte Phasen, d.h. mit einer Mischungslücke, vorliegen. Bevorzugt ist ein Temperaturbereich von 0°C bis 60°C und ein Druckbereich von 1*10⁵ bis 20*10⁵ Pa.

Das aus dem Extraktor (7) erhaltene Raffinat wird über Leitung (15) der Lösungsmittelstripperkolonne (11) zugeführt, wo über den Sumpf reines Wasser entnommen wird (Leitung (13)). Das Kopfprodukt der Lösungsmittelstripperkolonne wird einem Phasentrenner (9) zugeführt. Die dort anfallende wäßrige Phase geht über Leitung (10) in den Kopf der Lösungsmittelstripperkolonne (11) zurück, die organische Phase wird über Leitung (14) zum Extraktor (7) zurückgeführt.

Das aus dem Extraktor (7) abgezogene Extrakt, enthaltend Lösungsmittel, Essigsäure, Ameisensäure, Wasser und Schwersieder wird vom Extraktor (7) in eine Lösungsmitteldestillationskolonne (8) eingeleitet. In dieser. Kolonne wird,das Extrakt durch Destillation in drei Teilströme aufgeteilt.

Ein Teilstrom (Mischung (A)), bestehend aus einem Großteil des Lösungsmittels und Resten an Säure und Wasser wird dabei über Kopf der Kolonne entnommen und dem Phasentrenner (9) der Lösungsmittelstripperkolonne (11) eingeleitet. Alternativ kann dieser Teilstrom auch unter Umgehung des Phasentrenners direkt in den Extraktor (7) zurückgeleitet werden.

Ein weiterer Teilstrom (Mischung(B)), bestehend aus den Komponenten Wasser, Lösungsmittel und Ameisensäure, wird der Lösungsmitteldestillationskolonne (8) über einen Seitenabzug dampfförmig entnommen und einer Ameisensäuredestillationskolonne (4) zugeführt aus deren Sumpf über Leitung (19) reine Ameisensäure abgetrennt wird. Über Kopf wird der Kolonne (4) ein Gemisch aus Lösungsmittel und Wasser entnommen und über Leitung (16) dem Phasentrenner (9) zugeführt. Die organische Phase wird dort vom Wasser getrennt und über Leitung (14) in den Extraktor (7) zurückgeführt.
Der verbliebene Teilstrom (Mischung(C)), bestehend aus Essigsäure und Schwersiedern, wird über den Sumpf der Lösungsmitteldestillationskolonne (8) abgetrennt und einer Essigsäuredestillationskolonne (5) zur Auftrennung in reine Essigsäure und Schwersiedern zugeführt. Die Essigsäure wird über Kopf durch die Leitung (17) entnommen und die Schwersieder am Kolonnensumpf über Leitung (18) abgetrennt.

Bei der beschriebenen Ausführungsform kommt es durch die dampfförmige Entnahme des Feeds mittels eines Seitenabzugs an der Lösungsmittelkolonne (8) und der dampfförmigen Einspeisung desselben in die Ameisensäuredestillationskolonne zu einer Energieeinsparung gegenüber Systemen ohne eine derartige Ausführung.

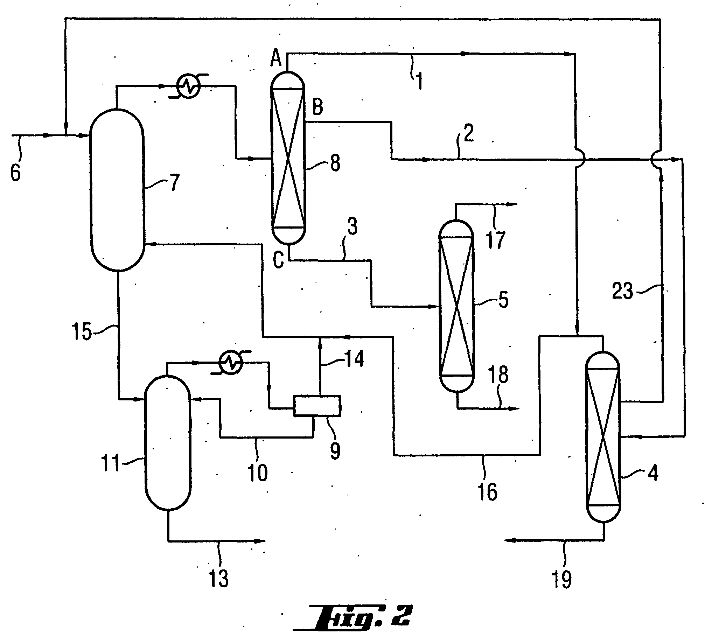

In einer besonderen Ausführungsform (Fig. 2) des oben. beschriebenen erfindungsgemäßen Verfahrens besitzt die Ameisensäuredestillationskolonne (4) einen zusätzlichen Seitenabzug. Aus diesem wird zur Erleichterung der Trennung ein kleiner Teilstron eines Gemisches enthaltend Wasser, Ameisensäure und Lösungsmittel entnommen und getrennt gesammelt oder über Leitung (23) zum Rohsäureeingang (6) oder eine andere Stelle des Extraktors (7) zurückgeführt. Am Sumpf der Kolonne (4) wird reine Ameisensäure über Leitung (19) entnommen. Das am Kopf der Ameisensäuredestillationskolonne (4) anfallende Gemisch, enthaltend Lösungsmittel, Wasser und Säurespuren wird über Leitung (16) in den Extraktor (7) zurückgeführt.

Ein besonderer Vorteil dieser Ausführungsform ist es, daß durch die Anbringung des Seitenabzugs an die Kolonne der Energiebedarf bei der Ameisensäuredestillation reduziert wird.

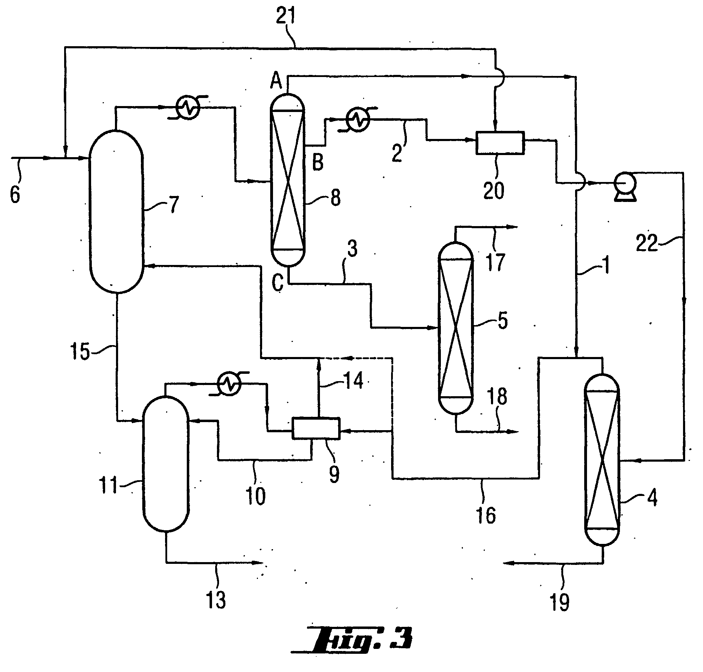

In einer weiteren besonderen Ausführungsform (Fig. 3) des erfindungsgemäßen Verfahrens wird der über einen Seitenabzug aus der Lösungsmittelkolonne (8) gasförmig entnommene Teilstrom (Mischung(B)), bestehend aus einem Gemisch aus Wasser, Lösungsmittel und Ameisensäure, vor der Einleitung in die Ameisensäuredestillationskolonne (4) kondensiert und dem Phasentrenner (20) zugeführt. Die abgetrennte organische Phase wird über Leitung (22) in die unter einem erhöhtem Druck von 1*10⁵ bis 10*10⁵ Pa arbeitende Ameisensäuredestillationskolonne (4) gepumpt. Am Sumpf dieser Kolonne wird über Leitung (19) reine Ameisensäure entnommen. Das am Kopf dieser Kolonne anfallende Gemisch enthaltend Lösungsmittel, Wasser und Säurespuren wird über Leitung (16) dem Phasentrenner (9) der Lösungsmittelstripperkolonne (11) zugeführt. Alternativ kann dieser Teilstrom auch unter Umgehung des Phasentrenners direkt in den Extraktor (7) zurückgeleitet werden. Die wäßrige Phase aus dem Phasentrenner (20) wird getrennt gesammelt oder über Leitung (21) zum Rohsäureeingang (6) oder eine andere Stelle des Extraktors (7) zurückgeführt.
Ein besonderer Vorteil dieser Ausführungsform liegt in einer weiteren Energieeinsparung. Durch die Kondensation des entnommenen Teilstroms (Mischung (B)) aus dem Seitenabzug der Lösungsmitteldestillationskolonne (8) und die anschließende Phasentrennung, kann ein Großteil des Wassers ohne energieaufwendige Destillation abgetrennt werden. Zusätzlich erleichtert ein höherer. Betriebsdruck und ein geringerer Wassergehalt im Feed der Ameisensäuredestillationskolonne (4) die Abtrennung der reinen Ameisensäure.

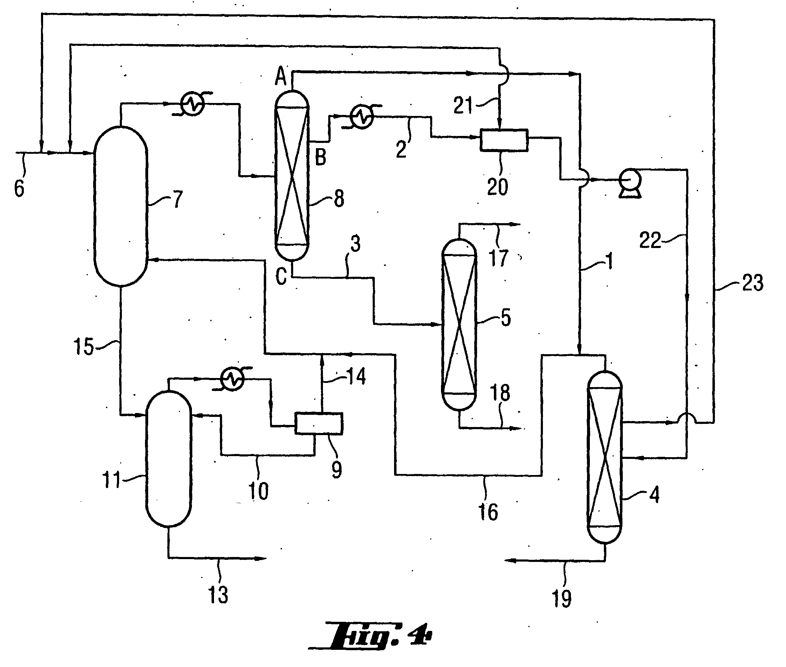

In einer weiteren besonderen Ausführungsform (Fig. 4) des erfindungsgemäßen Verfahrens wird der über einen Seitenabzug aus der Lösungsmittelkolonne (8) gasförmig entnommene Teilstrom (Mischung(B)), bestehend aus einem Gemisch aus Wasser, Lösungsmittel und Ameisensäure, vor der Einleitung in die Ameisensäuredestillationskolonne (4) kondensiert (analog Fig. 3) und dem Phasentrenner (20) zugeführt. Die entstehende organische Phase wird über Leitung (22) in die unter erhöhtem Druck bei 1*10⁵ bis 10*10⁵ Pa arbeitende Ameisensäuredestillationskolonne (4) gepumpt. Am Sumpf dieser Kolonne wird über Leitung (19) reine Ameisensäure entnommen. Das am Kopf dieser Kolonne anfallende Gemisch aus Lösungsmittel, Wasser und Säurespuren wird direkt über Leitung (16) zum Extraktor (7) zurückgeführt. Die Ameisensäuredestillationskolonne (4) besitzt in dieser Ausführungsform zusätzlich einen Seitenabzug (23). Dort wird zur Erleichterung der Trennung ein kleiner Teilstrom eines Gemisches enthaltend Wasser, Ameisensäure und Lösungsmittel entnommen und gesammelt oder zum Rohsäureeingang (6) oder eine andere Stelle des Extraktors (7) zurückgeführt. Die wäßrige Phase aus dem Phasentrenner (20) wird über Leitung (21) ebenfalls gesammelt oder zum Rohsäureeingang (6) oder eine andere Stelle des Extraktors (7) zurückgeführt.
Ein besonderer Vorteil dieser Ausführungsform ist es, daß durch die Anbringung des Seitenabzugs an die Kolonne (4) der Energiebedarf bei der Ameisensäuredestillation zusätzlich reduziert wird. Gleichzeitig wird durch die Kondensation des entnommenen Teilstroms (Mischung (B)) aus dem Seitenabzug der Lösungsmitteldestillationskolonne (8) und die anschließende Phasentrennung, ein Großteil des Wassers ohne energieaufwendige Destillation abgetrennt. Der geringere Wassergehalt im Feed der Ameisensäuredestillationskolonne (4) erleichtert die Abtrennung der reinen Ameisensäure zusätzlich.

In den folgenden Beispielen wird das erfindungsgemäße Verfahren unter Bezug auf die Abbildungen näher erläutert:

### Beispiel 1:

In einer erfindungsgemäßen Vorrichtung nach Fig. 1 wurde dem Extraktor (7) (Gegenstromextraktionskolonne mit stationärer Edelstahlpackung, organische Phase dispergiert) über Leitung (6) ein Rohsäurestrom aus 12,7 kg/h Essigsäure, 2,9 kg/h Ameisensäure, 49,7 kg/h Wasser und 0,2 kg/h Hochsieder zugeführt. Im stationären Zustand wurde der Lösungsmittelrückstrom über Leitung (14) zum Extraktor (7) so eingestellt, daß dieser 150 kg/h Methyltertiärbutylether (MTBE), 6 kg/h Wasser und 0,02 kg/h Ameisensäure enthielt. Der den Extraktor (7) verlassende Extraktstrom setzte sich aus 150 kg/h MTBE, 12,7 kg/h Essigsäure, 6,0 kg/h Wasser, 2,9 kg/h Ameisensäure und 0,2 kg/h Hochsieder zusammen.

Die Lösungsmitteldestillationskolonne (8) und die Essigsäurereinkolonne (5) wurden bei einem Druck von ca. 1*10⁵ Pa betrieben. Die Ameisensäurereinkolonne (4) wurden bei einem Druck von ca. 5*10⁵ Pa betrieben.

Am Sumpf der Lösungsmittelkolonne (8) wurde bei einer Temperatur von 121°C ein Teilstrom (Mischung (C)) über Leitung (3) abgeführt, der 12,7 kg/h Essigsäure und 0,2 kg/h Hochsieder enthielt. Aus dem Seitenabzug dieser Kolonne wurde ein Teilstrom (Mischung (B)) mit einer Temperatur von 84°C abgeführt, der aus 18,4 kg/h MTBE, 0,02 kg/h Essigsäure, 2,9 kg/h Ameisensäure und 3,0 kg/h Wasser bestand. Am Kopf dieser Kolonne wurde bei einer Temperatur von 56°C ein Teilstrom (Mischung (A)) abgeführt, der 131,6 kg/h MTBE, 3,0 kg/h Wasser und 0,01 kg/h Ameisensäure enthielt.

Am Sumpf der Essigsäurekolonne (5) wurde,bei einer Temperatur von 181,7 °C über Leitung (18) 0,2 kg/h Hochsieder entnommen. Am Kopf der Essigsäurekolonne (5) wurden bei einer Temperatur von 117,6 °C über Leitung (17) 12,7 kg/h Essigsäure entnommen.

Am Kopf der Ameisensäurereinkolonne (4) wurde bei einer Temperatur von 117 °C ein Strom aus 18,4 kg/h MTBE, 0,01 kg/h Ameisensäure und 3,0 kg/h Wasser entnommen. Am Sumpf der Ameisensäurekolonne (4) wurde bei einer Temperatur von 164 °C über Leitung (19) 0,02 kg/h Essigsäure und 2,9 kg/h Ameisensäure entnommen.

Um die Rohsäuremischung in 2,9 kg/h an 99,28 Gew.%-iger Ameisensäure und 12,7 kg/h an 99,99 Gew.%iger Essigsäure, aufzutrennen wurde ohne Feedvorwärmung vor den Destillationskolonnen folgender Energieeinsatz benötigt:
Sumpfheizung Lösungsmitteldestillationskolonne (8) : 35 kW
Sumpfheizung Ameisensäurekolonne (4) : 15 kW
Sumpfheizung Essigsäurereinkolonne (5) : 4,5 kW
Summe: 54,5 kW entspricht 3,5 kW pro kg Säure.

### Beispiel 2:

In einer Vorrichtung nach Ausführungsform Fig. 3 wurde dem Extraktor (7) (Gegenstromextraktionskolonne mit stationärer Edelstahlpackung, organische Phase dispergiert) über Leitung (6) ein Rohsäurestrom enthaltend 12,7 kg/h Essigsäure, 2,9 kg/h Ameisensäure, 47,8 kg/h Wasser und 0,2 kg/h Hochsieder zugeführt. Im stationären Zustand wurde der Lösungsmittelrückstrom über Leitung (14) zum Extraktor (7) so eingestellt, daß dieser 150,0 kg/h Methyltertiärbutylether (MTBE), 4,0 kg/h Wasser und 0,03 kg/h Ameisensäure enthielt. Der den Extraktor (7) verlassende Extraktstrom setzte sich aus 150,0 kg/h MTBE, 12,7 kg/h Essigsäure, 6,0 kg/h Wasser, 2,9 kg/h Ameisensäure und 0,2 kg/h Hochsieder zusammen.

Die Lösungsmitteldestillationskolonne (8) und die Essigsäurereinkolonne (5) wurden bei einem Druck von ca. 1*10⁵ Pa betrieben. Die Ameisensäurereinkolonne (4) wurde bei einem Druck von ca. 5*10⁵ Pa betrieben.

Am Sumpf der Lösungsmittelkolonne (8) wurde bei einer Temperatur von 121°C die Mischung (C), enthaltend 12,7 kg/h Essigsäure und 0,2 kg/h Hochsieder über Leitung (3) abgeführt. Aus dem Seitenabzug der Kolonne (8) wurde eine Mischung (B) bei einer Temperatur von 84°C abgeführt, die aus 18,4 kg/h MTBE, 0,02 kg/h Essigsäure, 2,9 kg/h Ameisensäure und 3,0 kg/h Wasser bestand. Am Kopf dieser Kolonne wurden bei einer Temperatur von 56°C eine Mischung (A) abgeführt, die aus 131,6 kg/h MTBE, 3,0 kg/h Wasser und 0,01 kg/h Ameisensäure bestand. Mischung (B) wurde kondensiert und über Leitung (2) in den Phasentrenner (20) geleitet. Die entstehenden Phasen wurden anschließend getrennt. Die organische Phase, enthaltend 18,2 kg/h MTBE, 1,0 kg/h Wasser, 0,01 kg/h Essigsäure und 2,5 kg/h Ameisensäure wurde über Leitung (22) abgeführt und als Feed in die Ameisensäurekolonne (4) eingespeist. Die wässrige Phase, enthaltend 2,0 kg/h Wasser, 0,01 kg/h Essigsäure und 0,4 kg/h Ameisensäure wurde getrennt gesammelt.

Am Sumpf der Essigsäurekolonne (5) wurden bei einer Temperatur 181,7 °C über Leitung (18) 0,2 kg/h Hochsieder entnommen. Am Kopf der Essigsäurekolonne (5) wurden bei einer Temperatur von. 117,6 °C über Leitung (17) 12,7 kg/h Essigsäure entnommen.

Am Kopf der Ameisensäurereinkolonne (4) wurde bei einer Temperatur von 115 °C über Leitung (16) ein Strom, enthaltend 18,2 kg/h MTBE, 0,02 kg/h Ameisensäure und 1,0 kg/h Wasser entnommen. Am Sumpf der Ameisensäurekolonne (4) wurden bei einer Temperatur von 164 °C über Leitung (19) 0.01 kg/h Essigsäure und 2,5 kg/h Ameisensäure entnommen.

Um die Rohsäuremischung in 2,5 kg/h an 99,65 Gew.-%iger Ameisensäure und 12,7 kg/h an 99,99 Gew.-%iger Essigsäure aufzutrennen wurde ohne Feedvorwärmung vor den Destillationskolonnen folgender Energieeinsatz benötigt: Sumpfheizung Lösungsmitteldestillationskolonne (8): 35 kW Sumpfheizung Ameisensäurekolonne (4): 7 kW Sumpfheizung Essigsäurereinkolonne (5): 4,5 kW Summe: 47 kW entspricht 3,1 kW pro kg Säure.

Eine Trennung des Rohsäuregemisches nach dem Stand der Technik, benötigt dagegen mindestens einen Energieeinsatz von 4 kW pro kg Säure und liefert eine verunreinigte (mit Wasser, Essigsäure, Schleppmittel z.B. Benzol oder chlorierte Kohlenwasserstoffe) Ameisensäure.

## Patentansprüche

1. Verfahren zur Trennung und Reinigung eines wäßrigen Gemisches aus den Hauptkomponenten Essigsäure, Ameisensäure und Schwersiedern durch Extraktion, mittels eines Lösungsmittels in einem Kreisverfahren, **dadurch gekennzeichnet, daß** der Raffinatstrom mit einem Großteil des Wassers einer Lösungsmittelstripperkolonne (11) zur Auskreisung des Wassers zugeführt wird und der Extraktstom in eine Lösungsmitteldestillationskolonne (8) geleitet wird, aus der in einem ersten Schritt über Kopf eine Mischung (A) mit einem Großteil des Lösungsmittels, aus einem Seitenabzug eine Mischung (B) bestehend aus Ameisensäure, Wasser und Lösungsmittel und über den Sumpf eine Mischung (C) bestehend aus Essigsäure und Hochsiedern abgetrennt werden, und zur weiteren Verarbeitung die Mischung (B) in eine Ameisensäuredestillationskolonne (4) übergeführt wird, und die Mischung (C) in eine Essigsäuredestillationskolonne (5) übergeführt wird, anschließend in der Essigsäuredestillationskolonne (5) über Kopf die reine Essigsäure isoliert wird, in der Ameisensäuredestillationskolonne (4) am Sumpf die reine. Ameisensäure isoliert wird und über Kopf ein Gemisch aus Lösungsmittel und Wasser abgezogen wird, welches zusammen mit der Mischung (A) nach Abtrennung des Wasseranteils, in den Extraktor (7) zurückgeführt wird.

2. Verfahren nach Anspruch 1, **dadurch gekennzeichnet, daß** der Extraktor ein- oder mehrstufig betrieben wird.

3. Verfahren nach Anspruch 1 bis 2, **dadurch gekennzeichnet, daß** der Lösungsmittelkreislauf im Extraktor im Gegenstrom zur Rohsäure verläuft.

4. Verfahren nach Anspruch 1 bis 3, **dadurch gekennzeichnet, daß** als Lösungsmittel gesättigte, ungesättigte und/oder cyclische Kohlenwasserstoffe mit 4 bis 8 Kohlenstoffatomen eingesetzt werden.

5. Verfahren nach Anspruch 1 bis 3, **dadurch gekennzeichnet, daß** als Lösungsmittel eine oder mehrere Verbindungen aus der Gruppe umfassend Ether, Ester, Ketone, Kohlenwasserstoffe und Alkohole eingesetzt werden.

6. Verfahren nach Anspruch 1 bis 5, **dadurch gekennzeichnet, daß** als Lösungsmittel eine oder mehrere Verbindungen aus der Gruppe umfassend Methyltertiärbutylether, Diisopropylether, Di-n-propylether, Ethylbutylether, Ethylacetat und Isopropylacetat verwendet werden.

7. Verfahren nach Anspruch 1 bis 6, **dadurch gekennzeichnet, daß** die Extraktion bei Temperaturen von 0 bis 60°C und Drücken von 1*10⁵ bis 20*10⁵ Pa durchgeführt wird.

8. Verfahren nach Anspruch 1 bis 7, **dadurch gekennzeichnet, daß** das Lösungsmittel in einem Mischungsverhältnis (Volumen/Volumen), zur Rohsäure zwischen den 0,5 bis 20-fachen vorliegt.

9. Verfahren nach Anspruch 1 bis 8, **dadurch gekennzeichnet, daß** die Ameisensäurekolonne (4) zusätzlich mit einem Seitenabzug ausgerüstet ist, aus dem ein Teilstrom entnommen wird.

10. Verfahren nach Anspruch 9, **dadurch gekennzeichnet, daß** der Teilstrom aus dem Seitenabzug der Ameisensäurekolonne in den Extraktor zurückgeführt wird.

11. Verfahren nach Anspruch 1 bis 10, **dadurch gekennzeichnet, daß** der über einen Seitenabzug aus der Lösungsmittelkolonne (8) gasförmig entnommene Teilstrom (Mischung(B)), bestehend aus einem Gemisch aus Wasser, Lösungsmittel und Ameisensäure, vor der Einleitung in die Ameisensäuredestillationskolonne (4) kondensiert und dem Phasentrenner (20) zugeführt wird, die entstehende organische Phase in die unter einem erhöhtem Druck arbeitende Ameisensäuredestillationskolonne (4) gepumpt wird, am Sumpf dieser Kolonne (4) reine Ameisensäure entnommen wird, das am Kopf dieser Kolonne (4) anfallende Gemisch, enthaltend Lösungsmittel, Wasser und Säurespuren dem Phasentrenner (9) der Lösungsmittelstripperkolonne (11) zugeführt wird.

12. Verfahren nach Anspruch 11, **dadurch gekennzeichnet, daß** die wäßrige Phase aus dem Phasentrenner (20) zum Extraktor (7) zurückgeführt wird.

## Claims

1. Process for the separation and purification of an aqueous mixture comprising the main components acetic acid, formic acid and high boilers by extraction with a solvent in a circulation process, **characterized in that** the raffinate stream containing a major part of the water is fed to a solvent stripping column (11) for removal of the water and the extract stream is coveyed to a solvent distillation column (8) from which, in a first step, a mixture (A) comprising the major part of the solvent is separated off via the top and a mixture (B) comprising formic acid, water and solvent is separated off at a side offtake and a mixture (C) comprising acetic acid and high boilers is separated off via the bottom, and, for further processing, the mixture (B) is fed to a formic acid distillation column (4) and the mixture (C) is fed to an acetic acid distillation column (5), the pure acetic acid is subsequently isolated at the top of the acetic acid distillation column (5), the pure formic acid is isolated at the bottom of the formic acid distillation column (4) and a mixture of solvent and water is taken off at the top and, together with the mixture (A) after separating off the water present, recirculated to the extractor (7).

2. Process according to Claim 1, **characterized in that** the extractor is operated in one or more stages.

3. Process according to either of claims 1 or 2, **characterized in that**. the solvent circuit in the extractor runs countercurrent to the crude acid.

4. Process according to any of claims 1 to 3, **characterized in that** the solvent used comprises saturated, unsaturated and/or cyclic hydrocarbons having from 4 to 8 carbon atoms.

5. Process according to any of claims 1 to 3, **characterized in that** the solvent used is one or more compounds selected from the group consisting of ethers, esters, ketones, hydrocarbons and alcohols.

6. Process according to any of claims 1 to 5, **characterized in that** the solvent used is one or more compounds selected from the group consisting of methyl tert-butyl ether, diisopropyl ether, di-n-propyl ether, ethyl butyl ether, ethyl acetate and isopropyl acetate.

7. Process according to any of claims 1 to 6, **characterized in that** the extraction is carried out at temperatures of 0 to 60°C and pressures of 1*10⁵ to 20*10⁵ Pa.

8. Process according to any of claims 1 to 7, **characterized in that** the mixing ratio (volume/volume) of solvent to crude acid is from 0.5 to 20.

9. Process according to any of claims 1 to 8, **characterized in that** the formic acid column (4) is additionally provided with a side offtake from which a substream is taken off.

10. Process according to Claim 9, **characterized in that** the substream from the side offtake of the formic acid column is recirculated to the extractor.

11. Process according to any of claims 1 to 10, **characterized in that** the substream (mixture (B)) comprising a mixture of water, solvent and formic acid taken off in gaseous form from the solvent column (8) via a side offtake is, prior to introduction into the formic acid distillation column (4), condensed and fed to the phase separator (20), and the organic phase formed is pumped into the formic acid distillation column (4) operated under superatmospheric pressure, pure formic acid is taken off at the bottom of this column (4) and the mixture comprising solvent, water and traces of acid obtained at the top of this column (4) is fed to the phase separator (9) of the solvent stripping column (11).

12. Process according to Claim 11, **characterized in that** the aqueous phase from the phase separator (20) is recirculated to the extractor (7).

## Revendications

1. Procédé pour la séparation et la purification par extraction d'un mélange aqueux constitué des composants principaux acide acétique, acide formique et substances à ébullition difficile, au moyen d'un solvant dans un procédé en circulation, **caractérisé en ce que** le flux de raffinat est introduit avec une grande partie de l'eau dans une colonne (11) de fractionnement de solvant pour éliminer l'eau du circuit et le flux extrait est guidé dans une colonne (8) de distillation du solvant, dont on sépare, dans une première étape, via la tête, un mélange (A) contenant une grande partie du solvant, via une évacuation latérale, un mélange (B) constitué d'acide formique, d'eau et de solvant et, via le fond de la colonne, un mélange (C) constitué d'acide acétique et de fractions à haut point d'ébullition, et, pour le traitement ultérieur, le mélange (B) est transféré dans une colonne (4) de distillation de l'acide formique et le mélange (C) dans une colonne (5) de distillation de l'acide acétique, l'acide acétique pur est ensuite isolé dans la colonne (5) de distillation de l'acide acétique via la tête, l'acide formique pur est isolé au niveau du fond de la colonne (4) de distillation de l'acide formique et un mélange de solvant et d'eau est soutiré via la tête, qui est recyclé avec le mélange (A), après séparation de la proportion d'eau, dans l'extracteur (7).

2. Procédé selon la revendication 1, **caractérisé en ce que** l'extracteur est opéré en une ou plusieurs étapes.

3. Procédé selon les revendications 1 à 2, **caractérisé en ce que** la circulation du solvant dans l'extracteur se fait à contre-courant par rapport à l'acide brut.

4. Procédé selon les revendications 1 à 3, **caractérisé en ce qu'**on utilise comme solvant des hydrocarbures saturés, insaturés et/ou cycliques comprenant 4 à 8 atomes de carbone.

5. Procédé selon les revendications 1 à 3, **caractérisé en ce qu'**on utilise comme solvant un ou plusieurs composés du groupe comprenant les éthers, les esters, les cétones, les hydrocarbures et les alcools.

6. Procédé selon les revendications 1 à 5, **caractérisé en ce qu'**on utilise comme solvant un ou plusieurs composés du groupe comprenant le méthyl-tert-butyléther, le diisopropyléther, le di-n-propyléther, l'éthylbutyléther, l'acétate d'éthyle et l'acétate d'isopropyle.

7. Procédé selon les revendications 1 à 6, **caractérisé en ce qu'**on réalise l'extraction à des températures de 0 à 60°C et des pressions de 1*10⁵ à 20*10⁵ Pa.

8. Procédé selon les revendications 1 à 7, **caractérisé en ce que** le solvant se trouve dans un rapport de mélange (volume/volume) entre 0,5 et 20 fois par rapport à l'acide brut.

9. Procédé selon les revendications 1 à 8, **caractérisé en ce que** la colonne (4) d'acide formique est de plus équipée d'une évacuation latérale dont un flux partiel est prélevé.

10. Procédé selon la revendication 9, **caractérisé en ce que** le flux partiel de l'évacuation latérale de la colonne d'acide formique est recyclé dans l'extracteur.

11. Procédé selon les revendications 1 à 10, **caractérisé en ce que** le flux partiel (mélange (B)) prélevé sous forme gazeuse via une évacuation latérale de la colonne (8) de solvant, constitué d'un mélange d'eau, de solvant et d'acide formique, est condensé avant l'introduction dans la colonne (4) de distillation d'acide formique et introduit dans le séparateur de phases (20), la phase organique qui se forme est pompée dans la colonne (4) de distillation de l'acide formique fonctionnant sous pression élevée, de l'acide formique pur est prélevé dans le fond de cette colonne (4), le mélange qui se forme en tête de cette colonne (4), contenant du solvant, de l'eau et des traces d'acide, est introduit dans le séparateur de phases (9) de la colonne (11) de fractionnement du solvant.

12. Procédé selon la revendication 11, **caractérisé en ce que** la phase aqueuse est recyclée du séparateur de phases (20) dans l'extracteur (7).
